(19) Europäisches Patentamt
European Patent Office
Office européen des brevets

(11) **EP 1 758 591 B2**

(12) **NOUVEAU FASCICULE DE BREVET EUROPEEN**
Après la procédure d'opposition

(45) Date de publication et mention de la décision concernant l'opposition:
**15.12.2010 Bulletin 2010/50**

(45) Mention de la délivrance du brevet:
**10.10.2007 Bulletin 2007/41**

(21) Numéro de dépôt: **05777134.7**

(22) Date de dépôt: **15.06.2005**

(51) Int Cl.:
*A61K 31/593* (2006.01)    *A61K 31/57* (2006.01)
*A61K 9/107* (2006.01)    *A61K 47/10* (2006.01)
*A61K 47/44* (2006.01)    *A61P 17/00* (2006.01)
*A61P 17/06* (2006.01)

(86) Numéro de dépôt international:
**PCT/FR2005/001495**

(87) Numéro de publication internationale:
**WO 2006/005843 (19.01.2006 Gazette 2006/03)**

(54) **COMPOSITION DE TYPE EMULSION INVERSE CONTENANT DU CALCITRIOL ET DU 17 PROPRIONATE DE CLOBETASOL, ET SON UTILISATION EN COSMETIQUE ET EN DERMATOLOGIE**

UMKEHREMULSIONSZUSAMMENSETZUNG MIT CALCITRIOL UND CLOBETASOL-17-PROPIONAT UND IHRE KOSMETISCHE UND DERMATOLOGISCHE VERWENDUNG

INVERT EMULSION COMPOSITION CONTAINING CALCITRIOL AND CLOBETASOL 17-PROPIONATE, AND USES THEREOF IN COSMETICS AND DERMATOLOGY

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**LV**

(30) Priorité: **17.06.2004 FR 0406612**

(43) Date de publication de la demande:
**07.03.2007 Bulletin 2007/10**

(73) Titulaire: Galderma S.A.
**6330 Cham (CH)**

(72) Inventeurs:
• ZANUTTO, Leslie
F-43230 Paulhaguet (FR)
• ORSONI, Sandrine,
Les Jardins de Mandelieu
F-06210 Mandelieu (FR)
• FREDON, Laurent,
Chemin du Camouyer
F-06330 Roquefort Les Pins (FR)

(74) Mandataire: Allab, Myriam et al
L'OREAL D.I.P.I.
25-29 Quai Aulagnier
92665 Asnières-sur-Seine (FR)

(56) Documents cités:
WO-A-00/64450          WO-A-03/011243
WO-A-2004/054588      WO-A-2004/069134
US-A- 4 610 978          US-A1- 2001 006 625

• LAMBA S ET AL: "Combination therapy with vitamin D analogues." BRITISH JOURNAL OF DERMATOLOGY, vol. 144, no. Supplement 58, avril 2001 (2001-04), pages 27-32, XP002253887 ISSN: 0007-0963
• AUSTAD J ET AL: "Clobetasol propionate followed by calcipotriol is superior to calcipotriol alone in topical treatment of psoriasis." JOURNAL OF THE EUROPEAN ACADEMY OF DERMATOLOGY AND VENEREOLOGY: JEADV. NETHERLANDS JUL 1998, vol. 11, no. 1, juillet 1998 (1998-07), pages 19-24, XP001154650 ISSN: 0926-9959

Remarques:
Le dossier contient des informations techniques présentées postérieurement au dépôt de la demande et ne figurant pas dans le présent fascicule.

**Description**

L'invention concerne une nouvelle composition de type émulsion inverse contenant deux actifs solubilisés, le calcitriol et le 17-propionate de clobétasol, ses utilisations en cosmétique et en dermatologie, ainsi que son procédé de préparation.

La peau humaine est constituée de deux compartiments, à savoir un compartiment profond, le derme, et un compartiment superficiel, l'épiderme.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale. On y trouve aussi des leucocytes, des mastocytes ou encore des macrophages tissulaires. Il contient également des vaisseaux sanguins et des fibres nerveuses.

L'épiderme est en contact avec l'environnement extérieur. Son rôle consiste à protéger l'organisme de la déshydratation et des agressions extérieures, qu'elles soient chimiques, mécaniques, physiques ou infectieuses.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Les cellules constituant l'épiderme sont délimitées par un domaine lipidique. Les lipides épidermiques sont synthétisés principalement dans l'épiderme vivant. Ils sont essentiellement constitués de phospholipides, de sphingolipides, de cholestérol, d'acides gras libres, de triglycérides, d'esters du cholestérol et d'alcanes. Au cours de la différenciation cellulaire, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides, constituants essentiels de la couche cornée de l'épiderme (stratum corneum).

Les lipides du ciment inter-cornéocytaire de la peau, et notamment les céramides, sont organisés en bi-couches lamellaires ou feuillets et participent à la cohésion du stratum corneum en vue de maintenir l'intégrité de la barrière et son rôle protecteur, antipénétration et anti-irritation notamment.

De nombreux actifs présentent la difficulté d'être très faiblement solubles dans les solvants cosmétiques ou pharmaceutiques couramment utilisés, notamment l'eau, et sensibles à un environnement aqueux. Cette sensibilité à l'eau peut conduire à une instabilité chimique de l'actif et/ou à une cristallisation de l'actif initialement solubilisé. Cette sensibilité à l'eau limite donc leur formulation dans des compositions cosmétiques ou dermatologiques appliquées par voie topique ou orale.

Les phénomènes de dégradation chimique et/ou de cristallisation de l'actif en présence d'eau ont pour conséquence une perte d'efficacité et une incertitude quant à la dose d'actif mise en oeuvre lors de son utilisation, ce qui va à l'encontre de l'objectif recherché. En outre, cette dégradation de l'actif et/ou sa cristallisation peuvent modifier la stabilité globale des compositions ainsi que leur aspect.

La forme galénique la plus couramment utilisée aujourd'hui en dermatologie est l'émulsion huile dans eau dans laquelle l'actif est préférentiellement solubilisé dans la phase lipophile. Mais cette solution reste peu satisfaisante car elle peut conduire à des produits peu agréables à utiliser, du à leur toucher collant, gras, instables physiquement.

Une autre possibilité est de solubiliser l'actif dans la phase hydrophile externe de l'émulsion, dans la limite de sa solubilité dans les milieux aqueux ou hydroglycoliques. Mais cette solution ne permet pas de résoudre les problèmes de stabilité chimique rencontrés, car l'activité en eau de l'émulsion reste très élevée.

La substitution de tout ou partie de la phase aqueuse par un ou plusieurs glycols conduirait à des formulations cosmétiquement peu acceptables. Il est en effet connu de l'homme du métier qu'au delà de 20% de glycol dans la phase externe, la formulation est cosmétiquement peu acceptable de par son toucher collant, et sa stabilité physique ne serait pas garantie.

L'élaboration d'une émulsion inverse (par émulsion inverse, on entend une émulsion de type : phase hydrophile dispersée dans phase lipophile) comme alternative n'était pas évidente pour l'homme du métier compte tenu des difficultés connues de formuler des actifs présentant des problèmes d'instabilité chimique et/ou de cristallisation dans l'eau. De plus la demanderesse a décrit dans les demandes de brevet WO 03/011243 et FR 2 850 575, des formulations glycol en silicone dont la viscosité s'est révélées parfois peu stables. En effet, le seuil d'écoulement de la formulation peut décroître en fonction du temps et de la température, comme la majorité des émulsions à phase continue huileuse. L'homme du métier n'était donc pas incité à utiliser ce type de composition pour résoudre le problème présent de formulation des deux actifs.

L'utilisation d'agents solubilisants hydrophiles comme le propylène glycol n'était également pas naturelle pour l'homme du métier compte tenu que les fortes concentrations nécessaires n'étaient pas favorables à une bonne stabilité physique de la formule et à un toucher cosmétique acceptable.

L'obtention d'une bonne tolérance avec des solubilisants comme le propylène glycol n'était également pas évidente car il a été montré chez l'homme des phénomènes d'intolérance cutanée, par exemple chez l'homme sain (Motoyoshi et al. Cosmet and toiletries, 99, 83-89, 1984).

Cependant, il est important pour la composition selon la présente invention d'incorporer une forte proportion de glycol afin de favoriser l'action du corticoide et conduire ainsi à une bonne vasoconstriction.

Par ailleurs, L'association de principes actifs n'est pas utilisée d'une manière classique dans le traitement des affections dermatologiques. Les difficultés principalement rencontrées par l'homme du métier lors de l'association de deux principes actifs sont les problèmes d'instabilité chimique et les interactions que les principes actifs peuvent présenter, lorsqu'ils sont présents au sein de la même formulation.

Le brevet WO 00/64450 a décrit l'association calcitriol-corticostéroïde, et notamment le 17 propionate de clobétasol, dans le traitement du psoriasis et de la dermatite séborrhéique. Ce brevet propose des compositions pharmaceutiques sous forme d'émulsion de type huile dans eau et eau dans huile. Cependant aucun exemple n'est décrit dans ce brevet associant les deux principes actifs. De même, le brevet WO2004/069134 décrit des émulsions de type inverse comprenant notamment du calcitriol, mais sans 17 propionate de clobétasol.

Peu de traitement existe donc associant le calcitriol et un corticoide. En effet, la vitamine D et ses dérivés sont instables dans les milieux aqueux, et sensibles aux pH acides alors que les corticoides et plus particulièrement le propionate de clobétasol, sont eux, sensibles aux milieux basiques. Il n'était donc pas évident pour l'homme du métier d'associer et de stabiliser au sein d'une même composition un actif de type vitamine D et un corticostéroide.

Le calcitriol est un analogue de la vitamine D utilisé pour réguler le taux de calcium dans l'organisme. Son utilisation dans le traitement des maladies dermatologiques a notamment été décrite dans le brevet US 4,610,978 pour le traitement du psoriasis. Ce brevet suggère des compositions comprenant du calcitriol qui peuvent en outre contenir une quantité d'un agent anti-inflammatoire tel qu'un corticostéroïde, cependant aucun mode de réalisation concret d'association de calcitriol et de corticostéroide n'est décrit ni testé en terme d'efficacité.

La demanderesse a décrit dans la demande FR 2 848 454 que l'association du calcitriol avec un corticostéroïde permet d'obtenir un effet synergique dans le traitement de certaines affections dermatologiques telles que le psoriasis, la dermatite atopique, la dermatite de contact et la dermatite séborrhéique, sans toutefois proposer des compositions pharmaceutiques stables associant les deux actifs.

Par ailleurs, dans le domaine de la dermatologie et de la formulation de compositions pharmaceutiques, l'homme du métier est amené à chercher des compositions qui, non seulement doivent être stables physiquement et chimiquement mais également doivent permettre de libérer l'actif et de favoriser sa pénétration à travers les couches cutanées afin d'en améliorer son efficacité.

Il existait donc un besoin d'une composition permettant de répondre à un ou plusieurs des aspects suivants : formuler deux actifs au sein d'une même composition, disposer d'une bonne stabilité de la formule au froid et à la chaleur, en particulier quant au maintien de la taille des globules et à l'absence de déphasage et notamment d'une bonne stabilité de la viscosité en fonction du temps, avoir une bonne résistance des actifs vis-à-vis des phénomènes d'oxydation, permettre une bonne stabilité chimique des actifs et une bonne disponibilité de ceux-ci pour la peau, présenter une bonne tolérance cutanée. Il est par ailleurs utile que la préparation de telles compositions bénéficie d'un mode de préparation avantageux.

Or la Demanderesse a mis au point de façon surprenante une formulation de type glycol dans huile qui permet de s'affranchir des différents problèmes liés aux aspects mentionnés ci-dessus en permettant notamment de disposer d'une bonne stabilité physique de la composition en tant que telle mais aussi de permettre une bonne stabilité chimique et disponibilité des actifs qu'elle contient. La composition selon l'invention a également l'avantage de présenter une bonne tolérance cutanée, d'autoriser une forte fraction volumique dispersée, et de présenter une forte teneur en glycol conduisant à une bonne vasoconstriction.

L'invention se rapporte donc à une composition contenant deux actifs solubilisés, un dérivé de vitamine D, à savoir le calcitriol et un corticoide, à savoir le 17-propionate de clobétasol (aussi appelé propionate de clobetasol par la suite). La composition selon l'invention est caractérisée en ce qu'elle est une composition contenant, en tant qu'actifs pharmaceutiques, du calcitriol et du 17-propionate de clobétasol, caractérisée en ce que la composition est une émulsion inverse contenant une phase hydrophile dispersée glycolique ou hydroglycolique, une phase continue lipophile et un émulsionnant de HLB compris entre 2 et 7.

ladite composition consistant en :

- 3 % de laurylméthicone copolyol ;
- 6 % de cyclométhicone-5 :
- 3 % de paraffine liquide ;
- 7 % de cétostéaryle isononanoate ;
- 0.025 % de 17-propionate de clobétasol ;
- 45.9347 % de propylène glycol ;
- 5 % de glycéryl polyméthacrylate et propylène glycol ;
- 14 % d'eau purifiée ;
- 8 % de sorbitol ;

- 3 % de magnésium sulfate heptahydrate ;
- 0,04 % de butylhydroxytoluène ;
- 0,0003 % de calcitriol ;
- 5 % d'éthanol 95 % ;

les pourcentages étant exprimés en poids par rapport au poids total de la composition.

Par le terme HLB, on entend le Rapport Hydrophile/Lipophile ou « Hydrophilic/Lipophilic Balance (HLB) » qui correspond à l'équilibre entre la dimension et la force du groupe hydrophile et la dimension et la force du groupe lipophile de l'émulsionnant.

L'invention permet également d'obtenir une bonne libération /pénétration de l'actif au niveau des différentes assises cutanées, conduisant à une bonne disponibilité de l'actif dans la peau, celui-ci étant utilisé sous forme solubilisée.

Par forme solubilisée, on entend une dispersion à l'état moléculaire dans un liquide, aucune cristallisation de l'actif n'étant visible à l'oeil nu ni même au microscope optique en polarisation croisée.

La présente invention consiste donc à préparer des émulsions inverses, contenant une phase hydrophile glycolique ou hydroglycolique, parfaitement stables (taille des globules et viscosité), même à forte fraction volumique dispersée, ne montrant aucune dégradation chimique et/ou cristallisation des actifs.

La présente invention divulgue également la préparation d'émulsions inverses contenant un actif solubilisé dans la phase lipophile de l'émulsion, et présentant une bonne stabilité physico-chimique, et aucune cristallisation de l'actif.

La présente invention concerne donc une composition contenant, en tant qu'actifs pharmaceutiques, du calcitriol et du 17-propionate de clobétasol, caractérisée en ce que la composition est une émulsion inverse contenant une phase hydrophile dispersée glycolique ou hydroglycolique, une phase continue lipophile et un émulsionnant de HLB compris entre 2 et 7.

ladite composition consistant en :

- 3 % de laurylméthicone copolyol ;
- 6 % de cyclométhicone-5 :
- 3 % de paraffine liquide ;
- 7 % de cétostéaryle isononanoate ;
- 0.025 % de 17-propionate de clobétasol ;
- 45.9347 % de propylène glycol ;
- 5 % de glycéryl polyméthacrylate et propylène glycol ;
- 14 % d'eau purifiée ;
- 8 % de sorbitol ;
- 3 % de magnésium sulfate heptahydrate ;
- 0,04 % de butylhydroxytoluène ;
- 0,0003 % de calcitriol ;
- 5 % d'éthanol 95 % ;

les pourcentages étant exprimés en poids par rapport au poids total de la composition.

La composition selon l'invention est de préférence adaptée à une application topique sur la peau, les phanères et/ou les muqueuses. Elle renferme généralement un milieu physiologiquement acceptable et une quantité de composé actif suffisante pour obtenir l'effet recherché.

Selon l'invention, le calcitriol est solubilisé dans un alcool. Par alcool utilisable en tant que solvant du calcitriol, on entend l'éthanol.

Selon l'invention, le glycol est le propylène glycol.

Les glycols auront avantageusement comme paramètre de solubilité un $\delta p$ inférieur à 10 étant entendu que les 3 paramètres de solubilité de Hansen : $\delta d$, $\delta p$ et $\delta h$ caractérisent, pour un constituant donné, les énergies correspondant respectivement aux interactions dispersives, polaires et de type liaisons hydrogène existant entre les molécules de ce constituant, $\delta p$ caractérisant plus particulièrement les forces d'interaction de Debye entre dipôles et étant fonction du nombre d'atomes d'oxygène dans la formule du constituant donné (S. paint Technology, 30, 195, 1967, « The three dimensional solubility parameter -Key to paint component affinities »).

De façon générale, comme composés lipophiles utilisables pour constituer une phase lipophile (ou grasse) continue des émulsions, on peut citer les huiles minérales (huile de paraffine), les huiles d'origine végétale (huile d'avocat, huile de soja), les huiles d'origine animale (lanoline), les huiles de synthèse (perhydrosqualène), les huiles siliconées (cyclométhicone, diméthicone) et les huiles fluorées (perfluoropolyéthers). On peut également utiliser des alcools gras tels que l'alcool cétylique, les alcools de Guerbet, en particulier l'octyldodecanol connu sous l'appelation Eutanol G, des acides gras, des cires, des gommes et en particulier les gommes de silicone.

Les composés constituant la phase lipophile de la composition selon l'invention sont l'huile de paraffine, l'isononanoate de cétéaryle commercialisé sous le nom de Cetiol SN et les cyclométhicones.

Il est également possible de caractériser un mode de réalisation préféré de la divulgation en se rapportant à l'activité en eau ($a_w$) de la phase hydrophile dans la composition selon l'invention.

L'invention se rapporte ainsi à une composition telle que définie précédemment, caractérisée en ce que l'activité en eau $a_w$ de la phase hydrophile est inférieure à 0,85.

L'activité en eau $a_w$ d'un milieu contenant de l'eau est le rapport de la pression de vapeur d'eau du produit « $P_{H2O}$ produit » et de la pression de vapeur de l'eau pure « $P_{H2O}$ pur » à la même température. Elle peut être exprimée aussi comme le rapport du nombre de molécules d'eau « $N_{H2O}$ » sur le nombre de molécules totales « $N_{H2O} + N_{corps}$ dissous », qui tient compte de celles des corps dissous « $N_{corps\ dissous}$ ».

Elle est donnée par les formules suivantes :

$$a_w = \frac{P_{H2O}\ produit}{P_{H2O}\ pur} = \frac{N_{H2O}}{N_{H2O} + N_{corps\ dissous}}$$

On peut utiliser différentes méthodes pour mesurer l'activité en eau $a_w$. La plus courante est la méthode manométrique par laquelle on mesure directement la pression de vapeur.

De manière classique, une composition cosmétique ou dermatologique a une activité en eau située autour de 0,95 à 0,99. Une activité en eau inférieure à 0,85 représente une diminution notable.

Pour la réalisation de l'émulsion inverse selon l'invention, la présence d'émulsionnants est obligatoire. Les émulsionnants (ou tensio-actifs ou surfactants) sont des substances naturelles ou synthétiques formées d'une partie hydrophile ou polaire et d'une partie lipophile ou apolaire. Ce sont des molécules amphiphiles puisqu'elles ont une double polarité. Les émulsionnants sont caractérisés par leur HLB ; si le HLB est élevé, la partie hydrophile est prédominante, si le HLB est faible, la partie lipophile prédomine.

L'émulsionnant selon l'invention permet de faire des émulsions inverses et a une HLB compris entre 2 et 7.

L'émulsionnant selon l'invention est le laurylméthicone copolyol vendu sous le nom d'Emulsifier 10 par la société DOW CORNING.

Selon l'invention, la composition comprend également des additifs dits rhéologiques. De façon surprenante, ces additifs, utilisés selon la présente invention, permettent d'obtenir la stabilité recherchée et notamment la stabilité de la viscosité dans le temps et à différentes températures.

L'additif rhéologique convenant à l'effet recherché, et appelé par la suite « stabilisateurs de viscosité » est :

- le Glyceryl polymethacrylate & propylène glycol vendu sous le nom commercial de Lubrajel CG par la société Sederma ;

La composition selon l'invention comprend un electrolyte. L'électrolyte utilisable selon l'invention est le Sulfate de Magnésium (Mg SO4).

La composition selon l'invention contient un anti-oxydant : le butylhydroxyanisole.

De façon connue, la composition de l'invention contient également le sorbitol.

La composition selon l'invention a un toucher cosmétiquement acceptable, une bonne tolérance cutanée, une bonne stabilité physique, c'est à dire absence de déphasage et maintien de la taille des globules au froid (4°C) et à la chaleur (45°C) sur une longue durée, par exemple sur 2 mois, avec notamment une viscosité stable sur cette période. La composition selon l'invention permet également de conférer aux actifs une bonne stabilité chimique et d'éviter sa cristallisation dans le temps.

De façon intéressante, la préparation de l'émulsion inverse simple selon l'invention s'est avérée ne nécessiter que peu d'énergie mécanique ou thermique par rapport aux préparations d'autres émulsions inverses déjà connues.

Il est donc également décrit le mode de préparation de la composition selon l'invention. En effet, la viscosité de la composition selon l'invention est fonction du mode opératoire. Le mode opératoire selon l'invention se compose de deux étapes critiques :

- La pré-émulsification : le glycol doit être incorporé lentement de manière à avoir la formation de petits globules, et la montée en vitesse de l'agitation doit être adaptée.

- L'émulsification proprement dite : la phase aqueuse doit être versée très lentement et sous agitation rayneri de 1000tr/mn.

Enfin l'agitation finale doit être très stricte et de 30mns de manière à être reproductible.

Le procédé de préparation des compositions selon l'invention comprend les étapes suivantes :

a) Préparation de la phase grasse A par :

- pesée des constituant de la phase grasse,
- chauffage à 55°C,
- homogénéisation ;

b) Préparation de la phase glycolique ou hydroglycolique B, avec incorporation du 17-propionate de clobetasol par :

- pesée du glycol et du 17-propionate de clobetasol,
- agitation magnétique jusqu'à solubilisation du corticoïde,
- chauffage à 55°C ;

c) Pré-Emulsification des deux phases précédemment préparées par :

- introduction lente de la phase glycolique B dans la phase grasse A,
- augmentation de la vitesse d'agitation au fur et à mesure de l'ajout du propylène glycol, à partir d'une vitesse initiale de 350tr/mn pour arriver à une vitesse de 1000tr/mn, vitesse finale qui sera constante pour le restant de la formulation.
- refroidissement de la pré-émulsion obtenue jusqu'à température ambiante ;

d) Préparation de la phase C, contenant les composés stabilisateurs de viscosité par:

- solubilisation de l'electrolyte à TA dans l'eau sous agitation,
- ajout du (des) stabilisateur(s) de viscosité,
- dispersion dans l'eau du (des) stabilisateur(s) de viscosité sous agitation magnétique ;

e) Ajout de la phase D, contenant le calcitriol par :

- préparation d'une solution mère du calcitriol dans le solvant,
- ajout d'un antioxydant,
- agitation jusqu'à solubilisation de l'actif ;

f) Mélange de la phase C et de la quantitié nécessaire de la phase D ; Par quantité nécessaire, on entend la quantité de solution mère permettant d'obtenir la quantité d'actif recherché dans la composition finale.
g) Emulsification du mélange obtenu à l'étape f) avec la pré-émulsion de l'étape c) par introduction très lente de la phase aqueuse C+D dans la pré-émulsion obtenue à l'étape c), sous agitation Rayneri à 1000 tr/mn.

Après la fin de l'émulsification, l'agitation est maintenue pendant 30mn.
L'invention couvre également l'utilisation de la nouvelle émulsion inverse telle que décrite précédemment en cosmétique et en dermatologie.
De part l'activité des composés utilisés dans la composition, la composition selon l'invention trouve une application dans la prévention et/ou le traitement des pathologies suivantes :

1) pour traiter les affections dermatologiques liées à un désordre de la différenciation ou de la prolifération des kératinocytes ou des sébocytes notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) pour traiter des troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal) ;
3) pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ;

4) pour traiter certaines affections inflammatoires cutanées ne présentant pas de trouble de la kératinisation, telles que l'eczéma atopique et les allergies de contact ;

5) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires ;

6) pour traiter d'autres désordres dermatologiques tels que les dermatoses bulleuses et les maladies du collagène ;

7) pour prévenir ou traiter les signes du vieillissement de la peau, qu'il soit photo-induit ou chronologique ou pour réduire les pigmentations et les kératoses actiniques, ou toutes pathologies cutanées associées au vieillissement chronologique ou actinique ;

8) pour prévenir ou traiter les troubles de la cicatrisation ou pour prévenir ou réparer les vergetures ;

9) pour lutter contre les troubles de la fonction sébacée tels que l'hyperséborrhée de l'acné ou la séborrhée simple ou encore l'eczéma séborrhéique ;

15) pour le traitement d'affections dermatologiques ou générales à composante immunologique ;

L'invention couvre également les préparations pharmaceutiques et les médicaments obtenus à partir des compositions selon l'invention.

En particulier, l'invention concerne donc l'utilisation d'une composition telle que définie précédemment pour la fabrication d'un médicament destiné au traitement du psoriasis.

L'invention sera maintenant illustrée par les exemples non limitatifs suivants.

**Exemple 1 : Stabilités du calcitriol dans différents excipients**

Des données de stabilités du calcitriol ont été générées dans divers excipients.

a) Stabilité du calcitriol dans l'éthanol

Solution de Calcitriol 30ppm dans qsp 100% d'éthanol absolu en présence de 0.02% de BHT.
Technique de dosage par HPLC contre substance de référence.
Au temps initial (T0), on considère que la composition comprend 100% de calcitriol.

Concentration mesurée de calcitriol en % par rapport à T0:

| Conditions de stabilité | T1 semaine | T2semaines | T3semaines | T4semaines |
|---|---|---|---|---|
| -18°C | 100.9% | 100.5% | 99.5% | 99.5% |
| +4°C | 97.7% | 98.6% | 98.1% | 97.7% |
| +30°C | / | 93.4% | / | 93.0% |

b) Stabilité du calcitriol dans le Miglyol 812 (caprilic/capric triglycerides)

Solution de Calcitriol 30ppm dans qsp 100% de Miglyol 812 en présence de 0.4% de BHT.
Technique de dosage par HPLC contre substance de référence.
Au temps initial (T0), on considère que la composition comprend 100% de calcitriol.

Concentration mesurée de calcitriol en % par rapport à T0 :

| Conditions de stabilité | T 2 semaines | T 4 semaines |
|---|---|---|
| +4°C | 98.3% | 105.2% |
| TA | 95.1% | 98.0% |
| +40°C | 91 % | 93.8% |

Ces résultats montrent une bonne stabilité du calcitriol dans le Miglyol 812

c) Stabilité du calcitriol dans le Cetiol SN (Cetearyl isononanoate)

Solution de Calcitriol 30ppm dans qsp 100% de Cetiol SN (Cetearyl isononanoate) en présence de 0.4% de BHT. Technique de dosage par HPLC contre substance de référence.
Au temps initial (T0), on considère que la composition comprend 100% de calcitriol.

Concentration mesurée de calcitriol en % par rapport à T0 :

| Conditions de stabilité | T2 semaines | T 4 semaines |
|---|---|---|
| +4°C | 98.6% | 98.1% |
| TA | 98.7% | 98.4% |
| +40°C | 99.0% | 98.9% |

Ces résultats montrent une bonne stabilité du calcitriol dans le Cétiol SN

**Exemple 2 : Stabilisation de la viscosité**

Le tableau suivant illustre que l'ajout d'additifs listés permet de stabiliser la viscosité dans le temps. Voici 3 exemples de formules suivies dans le temps :

| Dénomination chimique | Formule de référence | Formule 1 | Formule 2 | Formule 3 |
|---|---|---|---|---|
| Phase huileuse (mélange d'huiles) | 16 | 16 | 16 | 16 |
| Propylène glycol | QS 100% | QS 100% | QS 100% | QS 100% |
| Eau purifiée | 14 | 14 | 14 | 14 |
| Magnésium sulfate heptahydrate | 1 | 1 | 1 | 1 |
| Butylhydroxytoluène | 0.1 | 0.1 | 0.1 | 0.1 |
| Ethanol 95-96% | 5 | 5 | 5 | 5 |
| Lauryl methicone copolyol | 3 | | 3 | 3 |
| Cétéareth-20 | 1 | | | |
| Cyclopentasiloxane & PEG/PPG -19/19 diméthicone | | 4.5 | | |
| Silicone elastomer and décamethylcyclopentasiloxane | | 5 | | |
| PEG-150 Decylalcohol/SMDI Copolymère | | | 1 | |
| Glyceryl polymethacrylate and propylène glycol | | | | 5 |

Les valeurs des seuils d'écoulement sont données dans le tableau ci-après :

| température | Temps de mesure | Formule de référence | Formule N°1 | Formule N°2 | Formule N°3 |
|---|---|---|---|---|---|
| TA | 24h<br>1 mois<br>2 mois<br>3 mois | 78<br>81<br>69<br>50 | 58<br>47<br>/<br>60 | 38<br>/<br>43<br>39 | 52<br>49<br>49<br>NC |
| Conclusion | | Chute de viscosité | Stable | Stable | Stable |

Le seuil d'écoulement de la formule de référence chute dans le temps alors que les formules contenant des agents stabilisants sont parfaitement stables dans le temps.

Les additifs agissent par association en formant un réseau stabilisateur de l'émulsion.

## Exemple 3: Protocole de mesure de la stabilité des compositions selon l'invention

La stabilité physique des formulations est mesurée par une observation macroscopique et microscopique de la formulation à température ambiante (TA), à 4°C et à 40°C après 15j, 1 mois, 2 mois et 3 mois.

A TA, l'observation macroscopique permet de garantir l'intégrité physique des produits et l'observation microscopique permet de vérifier qu'il n'y a pas recristallisation de l'actif solubilisé.

A 4°C, l'observation microscopique vérifie la non-recristallisation des actifs solubilisés.

A 40°C, l'observation macroscopique vérifie l'intégrité du produit fini et l'observation microscopique permet de vérifier la stabilité de la taille des globules.

On complète la caractérisation du produit fini par une mesure du seuil d'écoulement.

On utilise un rhéomètre HAAKE de type VT550 avec un mobile de mesure SVDIN.

Les rhéogrammes sont réalisés à 25°C et à la vitesse de cisaillement de 4 $s^{-1}$ ($\gamma$), et en mesurant la contrainte de cisaillement. Par seuil d'écoulement ($\tau 0$ exprimé en Pascal) on entend la force nécessaire (contrainte de cisaillement minimum) pour vaincre les forces de cohésion de type Van der Waals et provoquer l'écoulement. Le seuil d'écoulement est assimilé à la valeur trouvée à la vitesse de cisaillement de $4s^{-1}$.

Ces mesures sont réalisées à T0, après 15 jours, 1, 2 et 3 mois.

Les exemples suivants sont des exemples de compositions de référence et d'une composition selon l'invention et leurs mesures de stabilité correspondantes.

## Exemple 4 : Composition de référence

| Matières premières | Quantités en % poids pour poids |
|---|---|
| Cyclopentasiloxane and PEG/PPG-19/19 dimethicone | 4.5 |
| Cyclomethicone 5 | 6 |
| Liquid paraffin | 3 |
| Cetostearyl isononanoate | 7 |
| CYCLOMETHICONE 5/DIMETHICONE CROSSPOLYMER | 5 |
| Clobetasol propionate | 0.001 |
| Propylene glycol | QSP 100 |
| Eau purifiée | 14 |
| Magnesium sulphate heptahydrate | 3 |
| Butylhydroxytoluene | 0.04 |
| Calcitriol | 0.0003 |
| Ethanol 95% | 5 |

## Exemple 5 : Composition de référence

| Matières premières | Quantités en % poids pour poids |
|---|---|
| Laurylmethicone copolyol | 3 |
| Cyclomethicone 5 | 6 |
| Liquid paraffin | 3 |
| Cetostearyl isononanoate | 7 |

(suite)

| Matières premières | Quantités en % poids pour poids |
|---|---|
| Clobetasol propionate | 0.05 |
| Propylene glycol | QSP 100 |
| Ethylène/propylene/butylenes/styrene copolymere dans l'isohexadecane | 5 |
| Eau purifiée | 14 |
| Magnesium sulphate heptahydrate | 3 |
| Butylhydroxytoluene | 0.04 |
| Calcitriol | 0.0009 |
| Ethanol 95% | 5 |

**Exemple 6 : Composition**

| Matières premières | Quantités en % poids pour poids |
|---|---|
| Laurylmethicone copolyol | 3 |
| Cyclomethicone 5 | 6 |
| Liquid paraffin | 3 |
| Cetostearyl isononanoate | 7 |
| Clobetasol propionate | 0.025 |
| Propylene glycol | QSP 100 |
| Glyceryl polymethacrylate and propylene glycol | 5 |
| Eau purifiée | 14 |
| Sorbitol | 8 |
| Magnesium sulphate heptahydrate | 3 |
| ButylHydroxytoluene | 0.04 |
| Calcitriol | 0.0003 |
| Ethanol 95% | 5 |

Mesures des stabilités de la composition selon le protocole de l'exemple 3

| SPECIFICATIONS A T0 | | | | |
|---|---|---|---|---|
| $\tau 0$ | | 152 | **Aspect macroscopique** | Aspect gel translucide, ferme et brillant. |
| **Centrifugation** | **3000tr/min** | RAS | **Aspect microscopique** | Emulsion très fine et homogène. |
| | **10000tr/min** | RAS | | |

EP 1 758 591 B2

|  |  |  | T15 J |
|---|---|---|---|
| TA | Macroscopie | | Conforme |
| | Microscopie | | Conforme |
| | $\tau 0$ | | 176 |
| | Centri | 3000 | RAS |
| | | 10000 | RAS |
| 4°C | Macroscopie | | Conforme |
| | Microscopie | | Conforme |
| 40° C | Macroscopie | | Conforme |
| | Microscopie | | |
| | $\tau$ 0 | | |

## Exemple 7 : Composition de référence

| Matières premières | Quantités en % poids pour poids |
|---|---|
| Laurylmethicone copolyol | 3 |
| Cyclomethicone 5 | 6 |
| Liquid paraffin | 3 |
| Cetostearyl isononanoate | 7 |
| Clobetasol propionate | 0.025 |
| Propylene glycol | QSP 100 |
| Glyceryl polymethacrylate and propylene glycol | 5 |
| Eau purifiée | 14 |
| Glycerol | 8 |
| Magnesium sulphate heptahydrate | 3 |
| ButylHydroxytoluene | 0.04 |
| Calcitriol | 0.0003 |
| Ethanol 95% | 5 |

## Exemple 8 de référence

| Matières premières | Quantités en % poids pour poids |
|---|---|
| Laurylmethicone copolyol | 3 |
| Cyclomethicone 5 | 6 |
| Liquid paraffin | 3 |
| Cetostearyl isononanoate | 7 |
| Clobetasol propionate | 0.025 |
| Propylene glycol | QSP 100 |

(suite)

| Matières premières | Quantités en % poids pour poids |
|---|---|
| Glyceryl polymethacrylate and propylene glycol | 5 |
| Eau purifiée | 14 |
| PEG-150/decylalcohol | 1 |
| Magensium sulphate heptahydrate | 3 |
| Butylhydroxytoluene | 0.04 |
| Calcitriol | 0.0003 |
| Ethanol 95% | 5 |

| SPECIFICATIONS A T0 | | | | |
|---|---|---|---|---|
| **Tau 0** | | 85 | **Aspect macroscopique** | Aspect gel translucide, brillant. |
| **Centrifugation** | **3000tr/min** | RAS | Emulsion très fine et homogène. | Emulsion très fine, pas de cristaux. |
| | **10000tr/mm** | RAS | | |

**Exemple 9: de référence**

| Matières premières | Quantités en % poids pour poids |
|---|---|
| Laurylmethicone copolyol | 3 |
| Cyclomethicone 5 | 5,5 |
| Liquid paraffin | 3 |
| Butylhydroxytoluene | 0.04 |
| Calcitriol | 0.0003 |
| Caprylic/capric triglyceride | 7,5 |
| Clobetasol propionate | 0.025 |
| Propylene glycol | QSP 100 |
| Glyceryl polymethacrylate and propylene glycol | 5 |
| Eau purifiée | 14 |
| PEG-150/decylalcohol | 1 |
| Magensium sulphate heptahydrate | 3 |
| Ethanol 95% | 5 |

| SPECIFICATIONS A T0 | | | | |
|---|---|---|---|---|
| **Tau 0** | | 82 | **Aspect macroscopique** | Aspect gel translucide, brillant |
| **Centrifugation** | **3000tr/mm** | RAS | Emulsion très fine et homogène. | Emulsion fine, pas de crstaux. |
| | **10000tr/min** | RAS | | |

**Exemple 10 : Etude de la libération / pénétration *in vitro* sur peau humaine de l'actif 17-propionate de clobétasol contenu dans 3 formulations différentes dont une selon l'invention.**

L'objectif est de quantifier la pénétration cutanée de l'actif formulé dans différentes formulations *in vitro* sur peau humaine après 16 heures d'application.

Formulations testées:

- Crème émolliente Temovate® à 0.05 % (w/w) de 17-propionate de clobétasol
- Crème Temovate® à 0.05 % (w/w) de 17-propionate de clobétasol
- Composition selon l'exemple 5 de référence

| Matières premières | Quantités en % poids pour poids |
|---|---|
| Laurylmethicone copolyol | 3 |
| Cyclomethicone 5 | 6 |
| Liquid paraffin | 3 |
| Cetostearyl isononanoate | 7 |
| Clobetasol propionate | 0.05 |
| Propylene glycol | QSP 100 |
| Ethylène/propylene/butylenes/styrene copolymere dans l'isohexadecane | 5 |
| Eau purifiée | 14 |
| Magnesium sulphate heptahydrate | 3 |
| Butylhydroxytoluene | 0.04 |
| Calcitriol | 0.0009 |
| Ethanol 95% | 5 |

La crème émolliente Temovate® est commercialisée par la société GLAXOSMITHKLINE.

**Conditions expérimentales** : L'absorption percutanée est évaluée grâce à des cellules de diffusion constituées de 2 compartiments séparés par la peau humaine. Les formulations ont été appliquées sans occlusion pendant un temps d'application de 16 heures. Les formulations ont été appliquées à raison de 10 mg de formulation par $cm^2$ (*i.e.* 10 microgrammes de clobétasol 17-propionate). Pendant la durée de l'étude, le derme est en contact avec un liquide récepteur non renouvelé en fonction du temps (mode statique). Les expériences ont été réalisées avec 3 échantillons de peau provenant de 3 donneurs différents. A la fin de la période d'application, l'excès de surface est enlevé et la distribution du 17-propionate de clobétasol est quantifiée dans les différents compartiments de la peau et dans le liquide récepteur. Les concentrations de 17-propionate de clobétasol ont été quantifiées en utilisant un méthode d'HPLC/MS/MS classiquement connu de l'homme de l'art. (LQ: 1 $ng.mL^{-1}$).

Les résultats sont exprimés en % de la dose appliquée (moyenne +/- écart-type) et sont consignés dans le tableau ci-dessous.

| Formulation | | Quantité totale ayant pénétré |
|---|---|---|
| Temovate crème émolliente | Mean<br>SEM | 5.00<br>1.34 |
| Temovate crème | Mean<br>SEM | 8.43<br>0.79 |
| Composition exemple 5 de référence | Mean<br>SEM | 12.15<br>1.29 |

Les résultats montrent que la quantité de clobétasol ayant pénétré avec la composition de référence est supérieure à celle des crèmes Temovate.

**Revendications**

1. Composition contentant, en tant qu'actifs pharmaceutiques, du calcitriol et du 17-propionate de clobétasol, **caractérisée en ce que** la composition est une émulsion inverse contenant une phase hydrophile dispersée glycolique ou hydroglycolique, une phase continue lipophile et un émulsionnant de HLB compris entre 2 et 7.
ladite composition consistant en :

   - 3 % de laurylméthicone copolyol ;
   - 6 % de cyclométhicone-5 ;
   - 3 % de paraffine liquide ;
   - 7 % de cétostéaryle isononanoate ;
   - 0.025 % de 17-propionate de clobétasol ;
   - 45,9347 % de propylène glycol ;
   - 5 % de glycéryl polyméthacrylate et propylène glycol ;
   - 14 % d'eau purifiée ;
   - 8 % de sorbitol ;
   - 3 % de magnésium sulfate heptahydrate ;
   - 0,04 % de butylhydroxytoluène ;
   - 0,0003 % de calcitriol ;
   - 5 % d'éthanol 95 % ;

   les pourcentages étant exprimés en poids par rapport au poids total de la composition.

2. Composition selon la revendication 1 à titre de médicament.

3. Utilisation d'une composition selon la revendication 1 pour la préparation d'un médicament destiné à la prévention ou au traitement :

   - des affections dermatologiques liées a un désordre de la différenciation ou de la prolifération des kératinocytes ou des sébocytes;
   - des troubles de la kératinisation;
   - des affections dermatologiques liées à un trouble de la kératinisation avec une composante inflammatoire et/ou immuno-allergique;
   - des affections inflammatoires cutanées ne pressentant pas de trouble de la kératinisation:
   - des proliférations dermiques ou épidermiques;
   - des désordres dermatologiques tels que les dermatoses bulleuses et les maladies du collagène;
   - des signes du vieillissement de la peau, qu'il soit photo-induit ou chronologique ou pour réduire les pigmentations et les kératoses actiniques, ou toutes pathologies cutanées associées au vieillissement chronologique ou actinique;
   - des troubles de la cicatrisation et des vergetures;
   - des troubles de la fonction sébacée tels que l'hyperseborrhée de l'acné ou la seborrhée simple ou encore l'eczema seborrhéique;
   - des affections dermatologiques à composante immunologique.

4. Utilisation selon la revendication 3, pour la préparation d'un médicament destiné à la prévention et au traitement du psoriasis.

**Claims**

1. Composition containing, as pharmaceutical active agents, calcitriol and clobetasol 17-propionate, **characterized in that** the composition is an inverse emulsion containing a glycol or water-glycol dispersed hydrophilic phase, a lipophilic continuous phase and an emulsifier with an HLB of between 2 and 7,
said composition comprising:

   - 3% of lauryl methicone copolyol;
   - 6% of cyclomethicone-5;
   - 3% of liquid paraffin;

- 7% of cetostearyl isononanoate;
- 0.025% of clobetasol 17-propionate;
- 45.9347% of propylene glycol;
- 5% of polyglyceryl methacrylate and propylene glycol;
- 14% of purified water;
- 8% of sorbitol;
- 3% of magnesium sulfate heptahydrate;
- 0.04% of butyl hydroxytoluene;
- 0.0003% of calcitriol;
- 5% of 95% ethanol;

the percentages being expressed on a weight basis relative to the total weight of the composition.

2. Composition according to Claim 1, as a medicament.

3. Use of a composition according to Claim 1 for the preparation of a medicament for preventing or treating:

- dermatological complaints associated with a differentiation or proliferation disorder of keratinocytes or sebocytes;
- keratinization disorders;
- dermatological complaints associated with a keratinization disorder with an inflammatory and/or immunoallergic component;
- cutaneous inflammatory complaints not showing a keratinization disorder;
- dermal or epidermal proliferations;
- dermatological disorders such as bullous dermatosis and collagen diseases;
- signs of ageing of the skin, whether photo-induced or chronological ageing, or for reducing actinic keratoses and pigmentations, or any cutaneous pathologies associated with chronological or actinic ageing;
- cicatrization disorders and stretch marks;
- sebaceous function disorders such as acneic hyperseborrhoea or simple seborrhoea or alternatively seborrhoeic eczema;
- dermatological complaints with an immunological component.

4. Use according to Claim 3 for the preparation of a medicament for preventing or treating psoriasis.

**Patentansprüche**

1. Zusammensetzung, die als pharmazeutische Wirkstoffe Calcitriol und Clobetasol-17-propionat enthält, **dadurch gekennzeichnet, dass** die Zusammensetzung eine inverse Emulsion ist, die eine dispergierte hydrophile Glycolphase oder Wasser/Glycolphase, eine kontinuierliche lipophile Phase und einen Emulgator mit einem HLB-Wert von 2 bis 7 enthält,
wobei die Zusammensetzung besteht aus:

• 3 % Laurylmethiconcopolyol;
• 6 % Cyclomethicone-5;
• 3 % flüssiges Paraffin;
• 7 % Cetostearylisononanoat;
• 0,025 % Clobetasol-17-propionat;
• 45,9347 % Propylenglycol;
• 5 % Glycerylpolymethacrylat und Propylenglycol;
• 14 % gereinigtes Wasser;
• 8 % Sorbitol;
• 3 % Magesiumsulfat-Heptahydrat;
• 0,04 % Butylhydroxytoluol;
• 0,0003 % Calcitriol;
• 5 % Ethanol 95%;

wobei die prozentualen Angaben in Gewichtsprozent, bezogen auf das Gesamtgewicht der Zusammensetzung, ausgedrückt sind.

2. Zusammensetzung nach Anspruch 1 als Arzneimittel.

3. Verwendung einer Zusammensetzung nach Anspruch 1 für die Herstellung eines Arzneimittels, das für die Vorbeugung oder Behandlung vorgesehen ist von:

- dermatologischen Erkrankungen, die mit einer Störung der Differenzierung oder Proliferation von Keratinocyten oder Sebocyten zusammenhängen;
- Verhornungsstörungen;
- dermatologischen Erkrankungen, die mit einer Keratinisierungsstörung mit entzündlicher und/oder immunoallergischer Komponente zusammenhängen;
- entzündlichen Hauterkrankungen, bei denen keine Verhornungsstörung vorliegt;
- Proliferationen der Dermis oder Epidermis;
- dermatologischen Erkrankungen, wie bullöse Dermatosen und Erkrankungen des Collagens;
- Anzeichen der Hautalterung, die lichtinduziert oder altersbedingt sein kann, oder um Pigmentierungen und aktinische Keratosen zu vermindern, oder allen Hauterkrankungen, die mit der altersbedingten oder aktinischen Alterung zusammenhängen;
- Störungen der Wundheilung und Dehnungsstreifen;
- Störungen der Talgdrüsenfunktion, wie Akne-Hyperseborrhoe oder Seborrhoe simplex oder seborrhoischen Ekzemen;
- dermatologischen Erkrankungen mit immunologischer Komponente.

4. Verwendung einer Zusammensetzung nach Anspruch 3 für die Herstellung eines Arzneimittels, das für die Vorbeugung und Behandlung von Psoriasis vorgesehen ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03011243 A
- FR 2850575
- WO 0064450 A
- WO 2004069134 A
- US 4610978 A
- FR 2848454

**Littérature non-brevet citée dans la description**

- **Motoyoshi et al.** *Cosmet and toiletries,* 1984, vol. 99, 83-89